(19) Europäisches Patentamt
European Patent Office
Office européen des brevets

(11) EP 2 863 861 B1

(12) **EUROPEAN PATENT SPECIFICATION**

(45) Date of publication and mention of the grant of the patent:
**18.10.2017 Bulletin 2017/42**

(21) Application number: **13730253.5**

(22) Date of filing: **21.06.2013**

(51) Int Cl.:
***A61K 8/02*** (2006.01) ***A61K 8/25*** (2006.01)
***A61Q 5/06*** (2006.01) ***A61Q 5/00*** (2006.01)

(86) International application number:
**PCT/EP2013/062961**

(87) International publication number:
**WO 2013/190078 (27.12.2013 Gazette 2013/52)**

# (54) COSMETIC COMPOSITION COMPRISING HYDROPHOBIC SILICA AEROGEL PARTICLES AND A FIXING POLYMER

KOSMETISCHE ZUSAMMENSETZUNGEN DIE PARTIKEL EINES HYDROPHOBEN SILIZIUM AEROGELS UND EIN FESTIGENDES POLYMER ENTHALTEN

COMPOSITION COSMÉTIQUE COMPRENANT DES PARTICULES D'AÉROGEL DE SILICE HYDROPHOBES ET UN POLYMÈRE DE FIXATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2012 FR 1255818**
**31.08.2012 US 201261695315 P**

(43) Date of publication of application:
**29.04.2015 Bulletin 2015/18**

(73) Proprietor: **L'Oréal**
**75008 Paris (FR)**

(72) Inventors:
- **BEBOT, Cécile**
**F-92600 Asnieres (FR)**
- **GRAS, Anne-sophie**
**F-75014 Paris (FR)**

(74) Representative: **Leray, Noelle**
**L'Oréal**
**Service DIPI**
**9 Rue Pierre Dreyfus**
**92110 Clichy (FR)**

(56) References cited:
**WO-A1-01/45651** **WO-A1-2007/051511**
**WO-A1-2010/054980** **WO-A2-2009/059869**

- **DOW CORNING: "Dow Corning VM-2270 Aerogel Fine particles", INTERNET CITATION, April 2009 (2009-04), pages 1-5, XP002650585, Retrieved from the Internet: URL:http://www2.dowcorning.com/DataFiles/090007c88020e235.pdf [retrieved on 2011-07-15]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 863 861 B1

## Description

The present invention relates to a cosmetic composition, as defined in the appended claims, comprising hydrophobic silica aerogel particles and at least one particular fixing polymer, and also to the use of such a composition for hair treatment, especially for the treatment of keratin fibres and in particular for fixing/shaping the hair.

In the field of hairstyling, in particular among the hair products intended for shaping and/or fixing the hairstyle, the hair compositions generally consist of a solution, usually an alcoholic or aqueous solution, and of one or more fixing polymers as a mixture with various cosmetic See e.g.WO2007051511 A1 and WO2009059869 A2. These compositions may in particular be in the form of hair gels, mousses or pastes that are generally applied to wet hair before blow-drying or drying.

However, compositions comprising fixing polymers are often tacky and can give the hair a certain greasy and coated feel.

There is therefore a real need to have a cosmetic composition that has good fixing and cosmetic properties, and that makes it possible to overcome the drawbacks mentioned above.

The applicant has discovered that, by combining hydrophilic silica aerogel particles and a hydrophilic fixing polymer, it is possible to obtain compositions that allow good fixing of the hairstyle and a clean feel, namely a natural feel, without a coated or greasy feel. Moreover, these compositions leave few or no residues on the head of hair after removal.

A subject of the present invention is therefore a cosmetic composition comprising hydrophobic silica aerogel particles and one or more hydrophilic fixing polymers.

The cosmetic composition obtained is easy to spread in the hands and on the hair, and provides body, texturizing and a clean feel, while at the same time providing good fixing.

The invention also relates to a method for the cosmetic treatment of keratin fibres, in particular for fixing and/or shaping keratin fibres, employing the cosmetic composition as defined above.

Another subject of the invention is the use of a composition as defined above for hair treatment, especially for treating keratin fibres and in particular for fixing and/or shaping the hair.

Other subjects, features, aspects and advantages of the invention will emerge even more clearly from reading the description and examples which follow.

The composition according to the invention comprises hydrophobic silica aerogel particles.

The composition used in the present invention thus comprises hydrophobic silica aerogel particles.

Aerogels are ultralight porous materials which were first produced by Kristler in 1932.

They are generally synthesized by a sol-gel process in a liquid medium and then dried by extraction with a supercritical fluid. The supercritical fluid most commonly used is supercritical $CO_2$. This type of drying makes it possible to avoid shrinkage of the pores and of the material.

Other types of drying also make it possible to obtain porous materials starting from gel, namely (i) drying by freeze drying, which consists in solidifying the gel at low temperature and in then subliming the solvent, and (ii) drying by evaporation. The materials thus obtained are referred to respectively as cryogels and xerogels. The sol-gel process and the various drying operations are described in detail in Brinker C.J. and Scherer G.W., Sol-Gel Science, New York, Academic Press, 1990.

The expression "hydrophobic silica" is understood to mean any silica, the surface of which is treated with silylating agents, for example halogenated silanes such as alkylchlorosilanes, siloxanes, in particular dimethylsiloxanes such as hexamethyldisiloxane, or silazanes, so as to functionalize the OH groups with Si-Rn silyl groups, for example trimethylsilyl groups. The hydrophobic aerogel particles that are used in the present invention must, at least, have a specific surface area per unit of mass (SM) ranging from 500 to 1500 $m^2/g$, preferably from 600 to 1200 $m^2/g$ and better still from 600 to 800 $m^2/g$.

Preferably, the hydrophobic aerogel particles that may be used in the present invention advantageously have an oil absorption capacity, measured at the wet point, ranging from 5 to 18 ml/g of particles, preferably from 6 to 15 ml/g and better still from 8 to 12 ml/g.

Preferably, the hydrophobic aerogel particles that may be used in the present invention advantageously have a size, expressed as the mean diameter (D[0.5]), of less than 1500 $\mu m$, preferably ranging from 1 to 30 $\mu m$, preferably from 5 to 25 $\mu m$, better still from 5 to 20 $\mu m$ and even better still from 5 to 15 $\mu m$.

The hydrophobic aerogel particles used in the present invention may advantageously have a tapped density $\rho$ ranging from 0.04 $g/cm^3$ to 0.10 $g/cm^3$ and preferably from 0.05 $g/cm^3$ to 0.08 $g/cm^3$.

Preferably, the hydrophobic aerogel particles used in the present invention have a specific surface area per unit of volume SV ranging from 5 to 60 $m^2/cm^3$, preferably from 10 to 50 $m^2/cm^3$ and better still from 15 to 40 $m^2/cm^3$.

According to one preferred embodiment, the hydrophobic aerogel particles according to the invention have a specific surface area per unit of mass (SM) ranging from 500 to 1500 $m^2/g$, preferably from 600 to 1200 $m^2/g$ and better still from 600 to 800 $m^2/g$, a size expressed as the mean diameter (D[0.5]) ranging from 1 to 30 $\mu m$ and/or an oil absorption

capacity measured at the wet point ranging from 5 to 18 ml/g of particles, preferably from 6 to 15 ml/g and better still from 8 to 12 ml/g.

According to another advantageous embodiment, the hydrophobic aerogel particles used in the present invention have a specific surface area per unit of mass (SM) ranging from 600 to 800 $m^2/g$ and a size, expressed as the volume mean diameter (D[0.5]), ranging from 5 to 20 $\mu m$ and better still from 5 to 15 $\mu m$.

The specific surface area per unit of mass can be determined by the nitrogen absorption method, known as the BET (Brunauer-Emmet-Teller) method, described in The Journal of the American Chemical Society, Vol. 60, page 309, February 1938, which corresponds to international standard ISO 5794/1 (appendix D). The BET specific surface area corresponds to the total specific surface area of the particles under consideration.

The absorption capacity measured at the wet point, denoted Wp, corresponds to the amount of oil which needs to be added to 100 g of particles in order to obtain a homogeneous paste.

It is measured according to the "wet point" method or the method for determining the oil uptake of a powder according to the principle described in standard NF T 30-022. It corresponds to the amount of oil adsorbed onto the available surface of the powder and/or absorbed by the powder by measuring the wet point, described below:

> An amount m = 2 g of powder is placed on a glass plate and the oil (isononyl isononanoate) is then added dropwise. After addition of 4 to 5 drops of oil to the powder, mixing is performed using a spatula, and addition of oil is continued until conglomerates of oil and powder have formed. From this point, the oil is added at the rate of one drop at a time and the mixture is subsequently triturated with the spatula. The addition of oil is stopped when a firm and smooth paste is obtained. This paste must be able to be spread over the glass plate without cracks or the formation of lumps. The volume Vs (expressed in ml) of oil used is then noted.

The oil uptake corresponds to the ratio Vs/m.

The sizes of the aerogel particles according to the invention can be measured by static light scattering using a commercial particle size analyser such as the MasterSizer 2000 machine from Malvern. The data are processed on the basis of the Mie scattering theory. This theory, which is exact for isotropic particles, makes it possible to determine, in the case of non-spherical particles, an "effective" particle diameter. This theory is described in particular in the publication by Van de Hulst, H.C., "Light Scattering by Small Particles", Chapters 9 and 10, Wiley, New York, 1957.

In the context of the present invention, this density can be assessed according to the following protocol, known as tapped density protocol:

> 40 g of powder are poured into a graduated measuring cylinder and then the measuring cylinder is placed on a Stav 2003 device from Stampf Volumeter. The measuring cylinder is subsequently subjected to a series of 2500 tapping actions (this operation is repeated until the difference in volume between two consecutive tests is less than 2%) and then the final volume Vf of tapped powder is measured directly on the measuring cylinder.

The tapped density is determined by the ratio: mass (m)/Vf, in this instance 40/Vf (Vf being expressed in $cm^3$ and m in g).

The specific surface area per unit of volume is given by the relationship:

$$SV = SM*\rho$$

where $\rho$ is the tapped density expressed in $g/cm^3$ and SM is the specific surface area per unit of mass expressed in $m^2/g$, as defined above.

The hydrophobic silica aerogel particles used according to the present invention are preferably silylated silica (INCI name: silica silylate) aerogel particles.

The preparation of hydrophobic silica aerogel particles modified at the surface by silylation is further described in document US 7 470 725.

Use will in particular be made of hydrophobic silica aerogel particles surface-modified with trimethylsilyl groups.

As hydrophobic silica aerogels that may be used in the invention, an example that may be mentioned is the aerogel sold under the name VM-2260 (INCI name: Silica silylate), by the company Dow Corning, the particles of which have a mean size of about 1000 microns and a specific surface area per unit of mass ranging from 600 to 800 $m^2/g$.

Mention may also be made of the aerogels sold by Cabot under the references Aerogel TLD 201, Aerogel OGD 201 and Aerogel TLD 203, Enova Aerogel MT 1100 and Enova Aerogel MT 1200.

Use will be made more particularly of the aerogel sold under the name VM-2270 (INCI name: Silica silylate), by the company Dow Corning, the particles of which have a mean size ranging from 5 to 15 microns and a specific

surface area per unit of mass ranging from 600 to 800 $m^2/g$.

The hydrophobic silica aerogel particles are used in a content ranging at least from 0.05% to 10% by weight, more preferentially from 0.1% to 5% by weight and even more preferentially from 0.2% to 3% by weight relative to the total weight of the composition containing them.

The composition according to the invention also comprises one or more hydrophilic fixing polymers.

For the purposes of the invention, the term "fixing polymer" is understood to mean any polymer that is capable, by application to the hair, of giving a shape to the head of hair or of making it possible to retain an already acquired shape.

More preferably, the hydrophilic fixing polymer is water-dispersible or water-soluble.

Even more preferentially, the fixing polymer is water-soluble.

For the purposes of the present invention, the expression "water-soluble or water-dispersible polymer" is understood to mean a polymer which, at pH 7 and 25°C, has a weight solubility in water great than or equal to 0.1 %, better still greater than or equal to 0.5% and even better still greater than or equal to 1%.

All the anionic, cationic, amphoteric and non-ionic hydrophilic fixing polymers and mixtures thereof used in the art may be used in the compositions according to the present application.

The anionic fixing polymers generally used are polymers comprising groups derived from carboxylic acid, sulfonic acid or phosphoric acid and have a number-average molecular weight of between approximately 500 and 5 000 000.

The carboxylic groups are provided by unsaturated monocarboxylic or dicarboxylic acid monomers such as those corresponding to the formula:

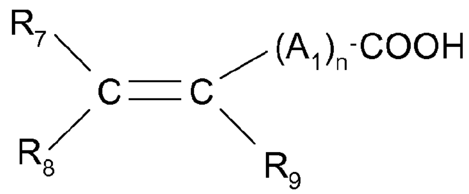

(I)

in which n is an integer from 0 to 10, $A_1$ denotes a methylene group optionally joined to the carbon atom of the unsaturated group or to the adjacent methylene group when n is greater than 1, via a heteroatom such as oxygen or sulfur, $R_7$ denotes a hydrogen atom or a phenyl or benzyl group, $R_8$ denotes a hydrogen atom or a lower alkyl or carboxyl group, and $R_9$ denotes a hydrogen atom, a lower alkyl group, or a $CH_2$-COOH, phenyl or benzyl group.

In the abovementioned formula, a lower alkyl group preferably denotes a group having 1 to 4 carbon atoms, and in particular the methyl and ethyl groups.

The anionic fixing polymers comprising carboxylic groups which are preferred according to the invention are:

A) copolymers of acrylic or methacrylic acid or salts thereof.
Among these polymers, mention may be made of copolymers of acrylic or methacrylic acid with a monoethylenic monomer such as ethylene, styrene, vinyl esters, acrylic or methacrylic acid esters, optionally grafted onto a polyalkylene glycol such as polyethylene glycol and optionally crosslinked. Such polymers are described in particular in French patent No.1 222 944 and German patent application No. 2 330 956, the copolymers of this type comprising an optionally N-alkylated and/or hydroxyalkylated acrylamide unit in their chain as described in particular in Luxembourg patent application Nos. 75370 and 75371. Mention may also be made of copolymers of acrylic acid and of $C_1$-$C_4$ alkyl methacrylate and terpolymers of vinylpyrrolidone, of acrylic acid and of $C_1$-$C_{20}$ alkyl methacrylate, for example lauryl methacrylate, such as the product sold by the company ISP under the name Acrylidone® LM (INCI name VP / acrylates / lauryl methacrylate copolymer), acrylic acid / ethyl acrylate / N-t-butylacrylamide terpolymers, such as the products Ultrahold® Strong and Ultrahold® 8 sold by the company BASF (INCI name Acrylates / t-butylacrylamide copolymer), methacrylic acid / ethyl acrylate / *tert*-butyl acrylate terpolymers, such as the products sold under the names Luvimer® 100 P or Luvimer® PRO 55 by the company BASF (INCI name Acrylates copolymer), copolymers of methacrylic acid and of ethyl acrylate, such as the products sold under the names Luvimer® MAE or Luviflex® Soft by the company BASF (INCI name Acrylates copolymer), acrylic acid / butyl acrylate / methyl méthacrylate terpolymers, such as the product sold under the name Balance® CR by the company Akzo Nobel (INCI name Acrylates copolymer), and the copolymers of methacrylic acid and of methyl methacrylate sold under the name Eudragit® L 100 by the company Rohm Pharma (INCI name Acrylates copolymer). Mention may also be made of branched block polymers containing (meth)acrylic acid monomers, such as the product sold under the name Fixate® G-100L by the company Lubrizol (INCI name AMP-acrylates / allyl methacrylate copolymer);

B) crotonic acid copolymers, such as those comprising vinyl acetate or propionate units in their chain and optionally other monomers such as allyl esters or methallyl esters, vinyl ether or vinyl ester of a linear or branched saturated carboxylic acid with a long hydrocarbon-based chain, such as those containing at least 5 carbon atoms, it being possible for these polymers optionally to be grafted or crosslinked, or alternatively another vinyl, allyl or methallyl ester monomer of an α- or β-cyclic carboxylic acid. Such polymers are described, *inter alia,* in French patent Nos. 1 222 944, 1 580 545, 2 265 782, 2 265 781, 1 564 110 and 2 439 798. Commercial products which fall into this category are the products Resyn® 28-2930 and 28-1310 sold by the company Akzo Nobel (INCI names VA / crotonates / vinyl decanoate copolymer and VA / crotonates copolymer, respectively). Mention may also be made of the products Luviset® CA 66 sold by the company BASF, Aristoflex® A60 sold by the company Clariant (INCI name VA / crotonates copolymer) and Mexomere® PW or PAM sold by the company Chimex (INCI name VA / vinyl butyl benzoate / crotonates copolymer);

C) copolymers of monounsaturated $C_4$-$C_8$ carboxylic acids or anhydrides selected from:

- copolymers comprising (i) one or more maleic, fumaric or itaconic acids or anhydrides and (ii) at least one monomer chosen from vinyl esters, vinyl ethers, vinyl halides, phenylvinyl derivatives, acrylic acid and its esters, the anhydride functions of these copolymers optionally being monoesterified or monoamidated. Such polymers are described, in particular, in US patent Nos. 2 047 398, 2 723 248 and 2 102 113, and GB patent No. 839 805. Commercial products are in particular those sold under the names Gantrez® AN or ES by the company ISP, such as Gantrez® ES 225 (INCI name Ethyl ester of PVM / MA copolymer) or Gantrez® ES 425L (INCI name Butyl ester of PVM / MA copolymer);
- copolymers comprising (i) one or more maleic, citraconic or itaconic anhydride units and (ii) one or more monomers chosen from allyl or methallyl esters optionally comprising one or more acrylamide, methacrylamide, α-olefin, acrylic or methacrylic ester, acrylic or methacrylic acid or vinylpyrrolidone groups in their chain,
  the anhydride functions of these copolymers optionally being monoesterified or monoamidated.

These polymers are described, for example, in French patent Nos. 2 350 384 and 2 357 241 by the applicant;

D) polyacrylamides comprising carboxylate groups.
The fixing polymers comprising units derived from sulfonic acid can be chosen from:

A') homopolymers and copolymers comprising vinylsulfonic, styrenesulfonic, naphthalenesulfonic or acrylamidoalkylsulfonic units.
These polymers can be chosen in particular from:

- polyvinylsulfonic acid salts having a molecular weight of approximately between 1000 and 100 000, and also the copolymers with an unsaturated comonomer such as acrylic or methacrylic acids and their esters, and also acrylamide or its derivatives, vinyl ethers and vinylpyrrolidone;
- polystyrenesulfonic acid salts such as the sodium salts that are sold for example under the name Flexan® II by Akzo Nobel (INCI name Sodium polystyrene sulfonate). These compounds are described in patent FR 2 198 719;
- polyacrylamidosulfonic acid salts, such as those mentioned in Patent US 4 128 631, and more particularly the polyacrylamidoethylpropanesulfonic acid, sold under the name Rheocare® HSP-1180 by Cognis (INCI name polyacrylamidomethylpropane sulfonic acid);

B') sulfonic polyesters, these polymers being advantageously obtained by polycondensation of at least one dicarboxylic acid, of at least one diol or of a mixture of diol and of diamine, and of at least one difunctional monomer comprising a sulfonic function. Among these polymers, mention may be made of:

- linear sulfonic polyesters such as those described in patent application Nos. US 3 734 874, US 3 779 993, US 4 119 680, US 4 300 580, US 4 973 656, US 5 660 816, US 5 662 893 and US 5 674 479. Such polymers are, for example, the products Eastman® AQ38S Polymer, Eastman® AQ55S Polymer and Eastman® AQ48 Ultra Polymer sold by the company Eastman Chemical (name Polyester-5) which are copolymers obtained from diethylene glycol, from 1,4-cyclohexanedimethanol, from isophthalic acid and from sulfoisophthalic acid salt;
- branched sulfonic polyesters such as those described in patent applications WO 95/18191, WO 97/08261 and WO 97/20899. Such compounds are, for example, the products Eastman® AQ10D Polymer (name

Polyester-13)

or Eastman® AQ1350 Polymer provided by the company Eastman Chemical (name Polyester-13).

According to the invention, the anionic fixing polymers are preferably chosen from copolymers of acrylic acid, such as the acrylic acid/ethyl acrylate/N-*tert*-butylacrylamide terpolymers sold in particular under the name Ultrahold® Strong by the company BASF, copolymers derived from crotonic acid, such as vinyl acetate/vinyl *tert*-butylbenzoate/crotonic acid terpolymers and the crotonic acid/vinyl acetate/vinyl neododecanoate terpolymers sold in particular under the name Resyn 28-2930 by the company Akzo Nobel, polymers derived from maleic, fumaric or itaconic acids or anhydrides with vinyl esters, vinyl ethers, vinyl halides, phenylvinyl derivatives and acrylic acid and esters thereof, such as the methyl vinyl ether/monoesterified maleic anhydride copolymers sold, for example, under the names Gantrez® ES 425L or ES 225 by the company ISP, the copolymers of methacrylic acid and of ethyl acrylate sold under the name Luvimer® MAE by the company BASF, and the vinyl acetate/crotonic acid copolymers sold under the name Luviset® CA 66 by the company BASF, and the vinyl acetate/crotonic acid copolymers grafted with polyethylene glycol sold under the name Aristoflex® A60 by the company Clariant, the vinylpyrrolidone/acrylic acid/lauryl methacrylate terpolymers sold under the name Acrylidone® LM by the company ISP, the polymer sold under the name Fixate® G-100L by the company Lubrizol, the vinyl acetate / crotonic acid/ vinyl p-*tert*-butylbenzoate copolymers sold under the names Mexomere® PW or PAM by the company Chimex.

The cationic fixing film-forming polymers that can be used according to the present invention are preferably selected from polymers comprising primary, secondary, tertiary and/or quaternary amine groups forming part of the polymer chain or directly attached thereto, and having a molecular weight of between 500 and about 5 000 000 and preferably between 1000 and 3 000 000.

Among these polymers, mention may be made more particularly of the following cationic polymers:

(1) homopolymers or copolymers derived from acrylic or methacrylic esters or amides and comprising at least one of the units of the following formulae:

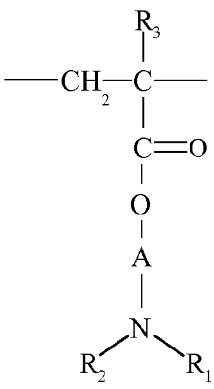 (A) ,

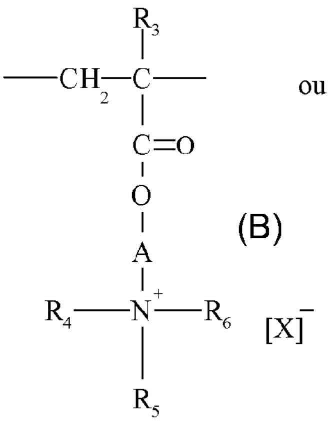 (B) ou

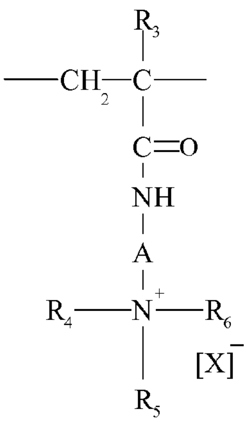 (C)

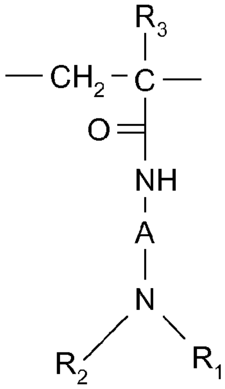 (D)

in which:

$R_3$ denotes a hydrogen atom or a $CH_3$ radical;
A is a linear or branched alkyl group comprising from 1 to 6 carbon atoms or a hydroxyalkyl group comprising from 1 to 4 carbon atoms;
$R_4$, $R_5$ and $R_6$, which may be identical or different, represent an alkyl group having from 1 to 18 carbon atoms or a benzyl radical;
$R_1$ and $R_2$, which may be identical or different, each represent a hydrogen atom or an alkyl group having from 1 to 6 carbon atoms;
X denotes a methosulfate anion or a halide such as chloride or bromide.

The copolymers of family (1) also contain one or more units derived from comonomers that may be chosen from the family of acrylamides, methacrylamides, diacetone acrylamides, acrylamides and methacrylamides substituted on the nitrogen with lower ($C_1$-$C_4$) alkyl groups, groups derived from acrylic or methacrylic acids or esters thereof, vinyllactams such as vinylpyrrolidone or vinylcaprolactam, and vinyl esters.
Thus, among these copolymers of family (1), mention may be made of:

- quaternized or non-quaternized vinylpyrrolidone/dialkylaminoalkyl acrylate or methacrylate copolymers, such as the products sold under the name Gafquat® by the company ISP, for instance Gafquat® 734 or Gafquat® 755 or Gafquat® 755N (INCI name Polyquaternium-11), or alternatively the products known as Copolymer® 845, 958 and 937 sold by ISP (INCI name VP/dimethylaminoethyl methacrylate copolymer). These polymers are described in detail in French patents 2 077 143 and 2 393 573,
- fatty-chain polymers containing a vinylpyrrolidone unit, such as the products sold under the name Styleze® W20L and Styleze® W10 by the company ISP (INCI name Polyquaternium-55),
- dimethylaminoethyl methacrylate/vinylcaprolactam/vinylpyrrolidone terpolymers, such as the products sold under the names Advantage HC 37 or Gaffix® VC 713 by the company ISP (INCI name Vinyl caprolactam / VP / dimethylaminoethyl methacrylate copolymer), and

- quaternized vinylpyrrolidone/dimethylaminopropylmethacrylamide copolymers, such as the products sold under the name Gafquat® HS 100 by the company ISP (name Polyquaternium-28);

(2) cationic guar gum derivatives, preferably containing quaternary ammonium, such as those described in US patents 3 589 578 and 4 031 307, such as guar gums containing trialkylammonium cationic groups. Such products are sold in particular under the trade names Jaguar® C13 S, Jaguar® C 15 and Jaguar® C 17 by the company Rhodia (INCI name Guar hydroxypropyltrimonium chloride);
(3) quaternary copolymers of vinylpyrrolidone and of vinylimidazole; mention may be made, for example, of vinylpyrrolidone / methylvinylimidazolium chloride copolymers, such as the products sold by the company BASF under the names Luviquat® FC550 or FC370, Luviquat® Excellence and Luviquat® Style (INCI name Polyquaternium-16), or vinylpyrrolidone / vinylimidazolium methosulfate / vinylcaprolactam terpolymers, such as the product Luviquat® Hold sold by the company BASF (INCI name Polyquaternium-46);
(4) chitosans or salts thereof; the salts that can be used are, in particular, chitosan acetate, lactate, glutamate, gluconate or pyrrolidonecarboxylate.
Among these compounds, mention may be made of the chitosan pyrrolidonecarboxylate sold under the name Kytamer® PC by the company Amerchol (INCI name Chitosan PCA);
(5) cationic cellulose derivatives such as copolymers of cellulose or of cellulose derivatives grafted with a water-soluble monomer comprising a quaternary ammonium, and described in particular in patent US 4 131 576, such as hydroxyalkylcelluloses, for instance hydroxymethyl-, hydroxyethyl- or hydroxypropylcelluloses grafted in particular with a methacryloyloxyethyltrimethylammonium, methacrylamidopropyltrimethylammonium or dimethyldiallylammonium salt.

The commercial products corresponding to this definition are, more particularly, the products sold under the name Celquat® L 200 and Celquat® H 100 by the company Akzo Nobel (INCI name Polyquaternium-4).
The amphoteric fixing polymers that may be used in accordance with the invention may be selected from polymers comprising units B and C distributed statistically in the polymer chain, where B denotes a unit derived from a monomer comprising at least one basic nitrogen atom and C denotes a unit derived from an acid monomer comprising one or more carboxylic or sulfonic groups, or alternatively B and C may denote groups derived from carboxybetaine or sulfobetaine zwitterionic monomers;
B and C can also denote a cationic polymer chain comprising primary, secondary, tertiary or quaternary amine groups, in which at least one of the amine groups bears a carboxylic or sulfonic group connected via a hydrocarbon-based group, or alternatively B and C form part of a chain of a polymer comprising an α,β-dicarboxylic ethylene unit in which one of the carboxylic groups has been made to react with a polyamine comprising one or more primary or secondary amine groups.

The amphoteric fixing polymers corresponding to the definition given above that are more particularly preferred are chosen from the following polymers:

(1) copolymers containing acidic vinyl units and basic vinyl units, such as those resulting from the copolymerization of a monomer derived from a vinyl compound bearing a carboxylic group such as, more particularly, acrylic acid, methacrylic acid, maleic acid, α-chloroacrylic acid, and of a basic monomer derived from a substituted vinyl compound containing at least one basic atom, such as, more particularly, dialkylaminoalkyl methacrylate and acrylate, dialkylaminoalkylmethacrylamide and acrylamide. Such compounds are described in US patent No. 3 836 537;

(2) polymers comprising units deriving from:

a) at least one monomer chosen from acrylamides or methacrylamides substituted on the nitrogen atom with an alkyl group,

b) at least one acidic comonomer containing one or more reactive carboxylic groups, and

c) at least one basic comonomer such as esters containing primary, secondary, tertiary and quaternary amine substituents of acrylic and methacrylic acids and the product of quaternization of dimethylaminoethyl methacrylate with dimethyl or diethyl sulfate.

The N-substituted acrylamides or methacrylamides that are more particularly preferred according to the invention are compounds in which the alkyl groups contain from 2 to 12 carbon atoms and more particularly N-ethylacrylamide, N-*tert*-butylacrylamide, N-*tert*-octylacrylamide, N-octylacrylamide, N-decylacrylamide, N-dodecylacrylamide and the corresponding methacrylamides.

The acidic comonomers are chosen more particularly from acrylic, methacrylic, crotonic, itaconic, maleic and fumaric acid and also alkyl monoesters, having 1 to 4 carbon atoms, of maleic or fumaric acid or anhydride.

The preferred basic comonomers are aminoethyl, butylaminoethyl, N,N'-dimethylaminoethyl and N-*tert*-butylaminoethyl methacrylates.

The copolymers of which the INCI name is Octylacrylamide/acrylates/butylaminoethyl methacrylate copolymer, such as the products sold under the names Amphomer®, Amphomer® LV71 or Balance® 47 by the company Akzo Nobel, are particularly used;

(3) crosslinked and acylated polyaminoamides partially or totally deriving from polyaminoamides of general formula:

$$-\!\!\left[CO-R_{10}-CO-Z\right]\!\!- \qquad (II)$$

in which $R_{10}$ represents a divalent group derived from a saturated dicarboxylic acid, a mono- or dicarboxylic aliphatic acid containing an ethylenic double bond, an ester of a lower alkanol having 1 to 6 carbon atoms of these acids, or a group derived from the addition of any one of said acids to a bis(primary) or bis(secondary) amine, and Z denotes a group derived from a bis(primary), mono(secondary) or bis(secondary) polyalkylene-polyamine and preferably represents:

a) in proportions of from 60 to 100 mol%, the group:

$$-NH-\left[(CH_2)_x-NH\right]_p- \qquad (IV)$$

where x = 2 and p = 2 or 3, or alternatively x = 3 and p = 2,

this group deriving from diethylenetriamine, from triethylenetetramine or from dipropylenetriamine;

b) in proportions of from 0 to 40 mol%, the group (IV) above in which x = 2 and p = 1 and which is derived from ethylenediamine, or the group deriving from piperazine:

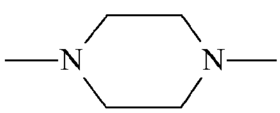

c) in proportions of from 0 to 20 mol%, the group $-NH-(CH_2)_6-NH-$ deriving from hexamethylenediamine, these polyaminoamides being crosslinked by addition reaction of a difunctional crosslinking agent chosen from epihalohydrins, diepoxides, dianhydrides and bis-unsaturated derivatives, using from 0.025 to 0.35 mol of crosslinking agent per amine group of the polyaminoamide and acylated by the action of acrylic acid, chloroacetic acid or an alkane sultone, or salts thereof.

The saturated carboxylic acids are preferably chosen from acids having 6 to 10 carbon atoms, such as adipic acid, 2,2,4-trimethyladipic acid and 2,4,4-trimethyladipic acid, terephthalic acid, acids containing an ethylenic double bond such as, for example, acrylic acid, methacrylic acid and itaconic acid.
The alkane sultones used in the acylation are preferably propane sultone or butane sultone; the salts of the acylating agents are preferably the sodium or potassium salts;
(4) polymers comprising zwitterionic units of formula:

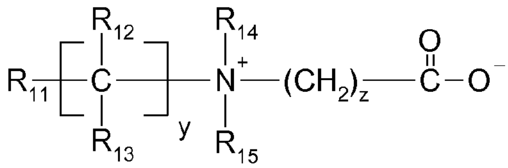 (IV)

in which $R_{11}$ denotes a polymerizable unsaturated group such as an acrylate, methacrylate, acrylamide or methacrylamide group, y and z represent an integer from 1 to 3, $R_{12}$ and $R_{13}$ represent a hydrogen atom, or a methyl, ethyl or propyl group, $R_{14}$ and $R_{15}$ represent a hydrogen atom or an alkyl group such that the sum of the carbon atoms in $R_{14}$ and $R_{15}$ does not exceed 10.
The polymers comprising such units may also comprise units derived from non-zwitterionic monomers such as dimethyl- or diethylaminoethyl acrylate or methacrylate or alkyl acrylates or methacrylates, acrylamides or methacrylamides or vinyl acetate.
Mention may be made, by way of example, of methyl methacrylate/methyl dimethylcarboxymethylammonioethyl methacrylate copolymers, such as the product sold under the name Diaformer Z-301N or Z-301W by the company Clariant (INCI name Acrylates copolymer);
(5) polymers derived from chitosan comprising monomer units corresponding to the following formulae:

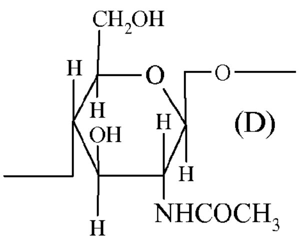 (D)

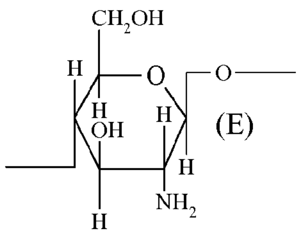 (E)

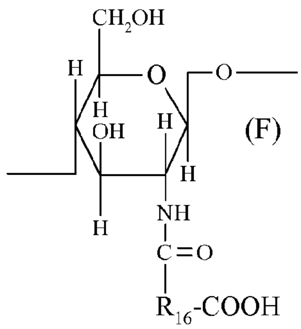 (F)

the unit (D) being present in proportions of between 0 and 30%, the unit (E) in proportions of between 5% and 50% and the unit (F) in proportions of between 30% and 90%, it being understood that, in this unit (F), $R_{16}$ represents a group of formula:

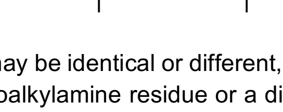

in which, if q = 0, $R_{17}$, $R_{18}$ and $R_{19}$, which may be identical or different, each represent a hydrogen atom, a methyl, hydroxyl, acetoxy or amino residue, a monoalkylamine residue or a dialkylamine residue that are optionally interspersed with one or more nitrogen atoms and/or optionally substituted with one or more amine, hydroxyl, carboxyl, alkylthio or sulfonic groups, or an alkylthio residue in which the alkyl group bears an amino residue, at least one of

the groups $R_{17}$, $R_{18}$ and $R_{19}$ being, in this case, a hydrogen atom;
or, if q = 1, $R_{17}$, $R_{18}$ and $R_{19}$ each represent a hydrogen atom, and also the salts formed by these compounds with bases or acids;
(6) polymers containing units corresponding to general formula (V) are described, for example, in French patent 1 400 366:

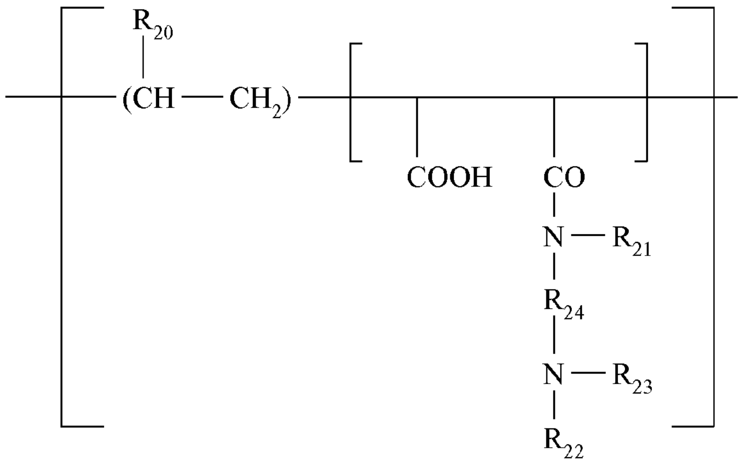

(V)

in which $R_{20}$ represents a hydrogen atom, a $CH_3O$, $CH_3CH_2O$ or phenyl group, $R_{21}$ denotes a hydrogen atom or a lower alkyl group such as methyl or ethyl, $R_{22}$ denotes a hydrogen atom or a $C_1$-$C_6$ lower alkyl group such as methyl or ethyl, $R_{23}$ denotes a $C_1$-$C_6$ lower alkyl group such as methyl or ethyl or a group corresponding to the formula: $-R_{24}-N(R_{22})_2$, $R_{24}$ representing a group $-CH_2-CH_2-$, $-CH_2-CH_2-CH_2-$ or $-CH_2-CH(CH_3)-$, $R_{22}$ having the meanings mentioned above;
(7) polymers derived from the N-carboxyalkylation of chitosan, such as N-carboxymethyl chitosan or N-carboxybutyl chitosan, for instance the product sold under the name Chitoglycan by the company Sinerga SPA (INCI name Carboxymethyl chitosan);
(8) amphoteric polymers of the -D-X-D-X type chosen from:

a) polymers obtained by the action of chloroacetic acid or sodium chloroacetate on compounds comprising at least one unit of formula:

-D-X-D-X-D- (VI)

where D denotes a group

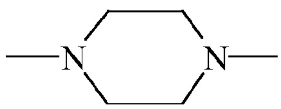

and X denotes the symbol E or E', E or E', which may be identical or different, denote a divalent group that is an alkylene group with a straight or branched chain containing up to 7 carbon atoms in the main chain, which is unsubstituted or substituted with hydroxyl groups and which can comprise, in addition to the oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups;
b) polymers of formula:

-D-X-D-X- (VI')

where D denotes a group

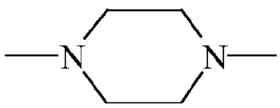

and X denotes the symbol E or E' and at least once E'; E having the meaning given above and E' is a divalent group that is an alkylene group with a straight or branched chain having up to 7 carbon atoms in the main chain, which is unsubstituted or substituted with one or more hydroxyl groups and containing one or more nitrogen atoms, the nitrogen atom being substituted with an alkyl chain that is optionally interrupted by an oxygen atom and necessarily comprising one or more carboxyl functions or one or more hydroxyl functions and betainized by reaction with chloroacetic acid or sodium chloroacetate;

(9) ($C_1$-$C_5$)alkyl vinyl ether/maleic anhydride copolymers partially modified by semiamidation with an N,N-dialkylaminoalkylamine such as N,N-dimethylaminopropylamine or by semiesterification with an N,N-dialkylaminoalkanol. These copolymers can also comprise other vinyl comonomers such as vinylcaprolactam.

Among the amphoteric fixing polymers mentioned above that are most particularly preferred according to the invention, mention will be made of those of family (3), such as the copolymers of which the INCI name is Octylacrylamide/acrylates/butylaminoethyl methacrylate copolymer, such as the products sold under the names Amphomer®, Amphomer® LV 71 or Balance® 47 by the company Akzo Nobel and those of family (4), such as the methyl methacrylate/methyl dimethylcarboxymethylammonioethyl methacrylate copolymers sold, for example, under the name Diaformer Z-301N or Z-301W by the company Clariant.
The non-ionic fixing polymers that may be used according to the present invention are chosen, for example, from:

- polyalkyloxazolines;
- vinyl acetate homopolymers;
- vinyl acetate copolymers, for instance copolymers of vinyl acetate and of acrylic ester; copolymers of vinyl acetate and of ethylene, or copolymers of vinyl acetate and of maleic ester, for example of dibutyl maleate;
- homopolymers and copolymers of acrylic esters, for instance copolymers of alkyl acrylates and of alkyl methacrylates, such as the products provided by the company Rohm GmbH under the name Eudragit® NE 30 D (INCI name Acrylates copolymer);
- copolymers of acrylonitrile and of a non-ionic monomer, chosen, for example, from butadiene and alkyl (meth)acrylates;
- styrene homopolymers;
- styrene copolymers, for instance copolymers of styrene, of alkyl acrylate and of alkyl methacrylate; copolymers of styrene and of butadiene, or copolymers of styrene, of butadiene and of vinylpyridine;
- polyamides;
- vinyllactam homopolymers, such as the vinylpyrrolidone homopolymers sold, for example, under the names Luviskol® K30 powder by the company BASF or PVP K30L or K60 solution or K90 by the company ISP, or such as the polyvinylcaprolactam sold under the name Luviskol® Plus by the company BASF (INCI name PVP);
- vinyllactam copolymers, such as a poly(vinylpyrrolidone/vinyllactam) copolymer sold under the trade name Luvitec® VPC 55K65W by the company BASF, poly(vinylpyrrolidone/vinyl acetate) copolymers, such as those sold under the name PVP/VA® S630L, E735, E635 and W735 by the company ISP, Luviskol® VA 73, VA 64 and VA 37 by the company BASF (INCI name VP/VA copolymer); and vinylpyrrolidone/methacrylamide/vinylimidazole terpolymers, for instance the product sold under the name Luviset® Clear by the company BASF (INCI name VP/methacrylamide/vinyl imidazole copolymer).

The alkyl groups of the abovementioned non-ionic polymers preferably have from 1 to 6 carbon atoms.
According to the invention, it is also possible to use fixing polymers of grafted silicone type comprising a polysiloxane portion and a portion constituted of a non-silicone organic chain, one of the two portions constituting the main chain of the polymer and the other being grafted to said main chain.
These polymers are described, for example, in patent applications EP-A-0 412 704, EP-A-0 412 707, EP-A-0 640 105 and WO 95/00578, EP-A-0 582 152 and WO 93/23009 and patents US 4 693 935, US 4 728 571 and US 4 972 037.
These polymers may be amphoteric, anionic or non-ionic, and are preferably anionic or non-ionic.
Such polymers are, for example, copolymers that may be obtained by free radical polymerization from the monomer mixture formed:

a) of 50% to 90% by weight of *tert*-butyl acrylate,

b) of 0% to 40% by weight of acrylic acid,

c) of 5% to 40% by weight of a silicone macromer of formula:

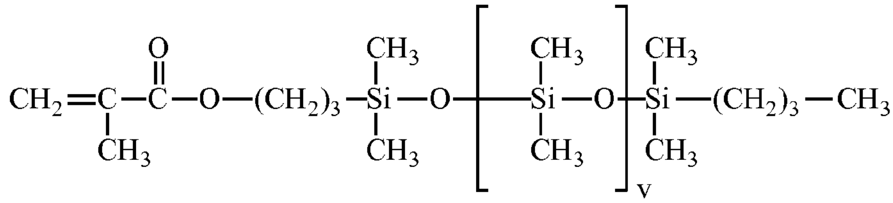

in which v is a number ranging from 5 to 700, the weight percentages being calculated relative to the total weight of the monomers.

Other examples of grafted silicone polymers are in particular polydimethylsiloxanes (PDMSs) to which mixed polymer units of the poly(meth)acrylic acid type and of the poly(alkyl (meth)acrylate) type are grafted via a thiopropylene-type connecting link and polydimethylsiloxanes (PDMSs) to which polymer units of the poly(isobutyl (meth)acrylate) type are grafted via a thiopropylene-type connecting link.

Grafted silicone polymers are, for example, sold under the names Silicone Plus Polymer® VS80 and VA70 by 3M (INCI names Polysilicone-8 and Polysilicone-7 respectively).

Another type of silicone fixing polymer that may be mentioned is the product Luviflex® Silk sold by BASF (INCI name PEG/PPG-25/25 dimethicone/acrylates Copolymer).

As fixing polymers, it is also possible to use functionalized or non-functionalized, cationic, non-ionic, anionic or amphoteric, silicone or non-silicone polyurethanes, or mixtures thereof.

The polyurethanes particularly intended by the present invention are those described in patent applications EP 0 751 162, EP 0 637 600, EP 0 648 485 and FR 2 743 297, of which the Applicant is the Proprietor, and patent applications EP 0 656 021 and WO 94/03510 from the company BASF and EP 0 619 111 from the company National Starch.

Mention may be made, as polyurethanes particularly suitable in the present invention, of the products sold under the names Luviset PUR® and Luviset® Si PUR by the company BASF (INCI names Polyurethane-1 and Polyurethane-6 respectively).

In one preferred variant, the hydrophilic fixing polymers are non-ionic, cationic or anionic.

In another preferred variant, the fixing polymers are soluble in the composition of the invention.

The concentration of hydrophilic fixing polymer(s) used in the compositions according to the present invention may range from 0.1% to 20%, preferably from 0.5% to 10% and even more preferentially from 0.5% to 8% by weight relative to the total weight of the composition.

The composition according to the invention may also comprise one or more thickeners that may be chosen from natural or synthetic, associative or non-associative polymeric thickeners and non-polymeric thickeners.

Examples of polymeric thickeners that may be mentioned include cellulose thickeners, for example hydroxyethyl cellulose, hydroxypropyl cellulose and carboxymethyl cellulose, guar gum and its derivatives, for example hydroxypropyl guar, sold by the company Rhodia under the reference Jaguar HP 105, gums of microbial origin, such as xanthan gum and scleroglucan gum, carrageenan, for example the carrageenan powder sold by the company Cargill under the reference Satiagum UTC 30, synthetic polymeric thickeners, resulting from radical polymerization reactions or polycondensation reactions such as crosslinked homopolymers of acrylic acid or of acrylamidopropanesulfonic acid, for example Carbomer, or non-ionic, anionic or amphoteric associative polymers, such as the polymers sold under the names Pemulen TR1 or TR2 by the company Goodrich, Salcare SC90 by the company Allied Colloids, Aculyn 22, 28, 33, 44 or 46 by the company Rohm & Haas and Elfacos T210 and T212 by the company Akzo or else sodium polyacrylate such as the product sold by the company Sensient under the commercial reference Covacryl MV 60.

When the composition comprises thickener(s), the thickener(s), preferably polymeric thickener(s), is (are) present in a content ranging from 0.1% to 10% by weight, preferably in a content ranging from 0.2% to 5% by weight, relative to the total weight of the composition.

Preferably, the composition comprises water, preferably in a content of greater than or equal to 5% by weight relative to the total weight of the composition. The water content preferentially ranges from 5% to 98%, preferably from 30% to 95%, better still from 50% to 95% and even better still from 70% to 95% by weight relative to the total weight of the composition.

Preferably, the composition of the invention comprises less than 5% of fatty substances and more preferably less than 3% of fatty substances. In one preferred variant of the invention, it does not comprise fatty substances other than an optional propellant

For the purposes of the invention, the term "fatty substance" is understood to mean an organic compound that

is insoluble in water at ordinary temperature (25°C) and at atmospheric pressure (760 mmHg) (solubility of less than 5%, preferably than 1% and even more preferentially than 0.1 %). They have in their structure at least one hydrocarbon-based chain containing at least 6 carbon atoms or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

The fatty substances do not contain salified or unsalified carboxylic acid groups (COOH or COO-).

The fatty substances according to the invention are neither (poly)oxyalkylenated nor (poly)glycerolated.

The composition may also comprise one or more water-soluble liquid organic solvents preferably chosen from monoalcohols, such as ethanol or isopropanol; polyols, such as propylene glycol or glycerol; polyol ethers; and mixtures thereof.

The composition according to the invention may comprise a propellant. For example, mention may be made of liquefied gases such as dimethyl ether, 1,1-difluoroethane, or $C_{3-5}$ alkanes, for instance propane, isopropane, n-butane, isobutane or pentane, or compressed gases such as air, nitrogen or carbon dioxide, and mixtures thereof.

Mention may be made preferentially of $C_{3-5}$ alkanes and in particular propane, n-butane and isobutane, and mixtures thereof.

When it comprises propellant(s), the composition comprises one or more propellant(s) in an amount ranging from 1% to 60% by weight, better still from 2% to 50% by weight and even more preferentially from 4% to 40% by weight relative to the total weight of the composition.

The composition for fixing and/or shaping the hair according to the invention may also contain one or more additives chosen from surfactants, conditioning agents, vitamins and provitamins including panthenol, sunscreens, pearlescent agents and opacifiers, dyes, sequestrants, plasticizers, solubilizers, acidifying agents, basifying agents, neutralizers, antioxidants, antifoams, moisturizers, emollients, hydroxy acids, penetrants, fragrances, preservatives and solid particles and fillers different from the aerogels, such as, for example, coloured or colourless mineral and organic pigments.

These additives may be present in the composition according to the invention in an amount ranging from 0 to 20% by weight relative to the total weight of the composition.

Of course, those skilled in the art will take care to choose the optional additional compounds and/or the amount thereof such that the advantageous properties of the compositions used according to the invention are not, or not substantially, adversely affected by the envisaged addition.

The composition according to the invention may be, *inter alia,* in the form of liquids that are thickened to a greater or lesser degree, gels, sera, creams, pastes, sprays or mousses.

In particular, the composition of the invention may be applied using an aerosol device.

Preferably, the composition according to the invention is in the form of gels or pastes.

The cosmetic composition according to the invention may advantageously be used for the cosmetic treatment of the hair. In particular, the composition may be used for styling the hair, for example for shaping and/or fixing the hair.

The present invention also relates to a method for the cosmetic treatment of the hair, for example a method for shaping and/or fixing the hairstyle, which consists in applying, to the hair, an effective amount of a composition according to the invention as described above and then in carrying out an optional rinsing after an optional leave-on time.

Preferably, the composition according to the invention is not rinsed off.

The method of the invention may be performed at ambient temperature (25°C) or using heat at a temperature ranging from 40 to 230°C using any heating device: hood, hairdryer, tongs.

The invention is illustrated in more detail in the following examples, which are provided by way of illustration and without limitation of the invention.

## EXAMPLES

The hairstyling paste compositions were prepared with the following ingredients:

Example 1:

| **Chemical name** | **% AM** |
|---|---|
| Trimethylated silica (1) | 1.96 |
| Crosslinked carboxyvinyl homopolymer(2) | 1.8 |
| Butyl acrylate/acrylic acid/methacrylic acid branched block polymer(3) | 1 |
| Triethanolamine | 0.8 |
| Phenoxyethanol | 1 |

(continued)

| Chemical name | % AM |
|---|---|
| Water | Qs 100 |
| (1) Aerogel VM2270 (DOW CORNING)<br>(2) Ultrez 10 (NOVEON)<br>(3) Fixate G100 (LUBRIZOL) | |

Example 2:

| Chemical name | % AM |
|---|---|
| Trimethylated silica (1) | 1.96 |
| Carboxyvinyl polymer(3) | 0.8 |
| Vinylpyrrolidone/vinyl acetate copolymer(4) | 2.5 |
| Triethanolamine | 0.8 |
| Phenoxyethanol | 1 |
| Water | Qs 100 |
| (1) Aerogel VM2270 (DOW CORNING)<br>(3) Synthalen K (3V)<br>(4) Luviskol VA 64W (BASF) | |

Example 3:

| Chemical name | % AM |
|---|---|
| Trimethylated silica (1) | 1.96 |
| Acrylic polymer (5) | 0.7 |
| Vinylpyrrolidone/dimethylaminoethyl methacrylate copolymer(6) | 0.96 |
| Triethanolamine | 0.8 |
| Phenoxyethanol | 1 |
| Water | Qs 100 |
| (1) Aerogel VM2270 (DOW CORNING)<br>(5) Ultrez 21 (NOVEON)<br>(6) Copolymer 845 (ISP) | |

Example 4:

| Chemical name | % AM |
|---|---|
| Trimethylated silica (1) | 1.96 |
| Acrylates/C10-30 alkyl acrylate crosspolymer(7) | 1.4 |
| Vinylpyrrolidone/vinyl acetate copolymer(4) | 2.5 |
| Vinylpyrrolidone/dimethylaminoethyl methacrylate copolymer(6) | 0.96 |
| Triethanolamine | 0.8 |
| Phenoxyethanol | 1 |

(continued)

| Chemical name | % AM |
|---|---|
| Water | Qs 100 |
| (1) Aerogel VM2270 (DOW CORNING)<br>(4) Luviskol VA 64W (BASF)<br>(6) Copolymer 845 (ISP)<br>(7) Pemulen TR-1 (NOVEON) | |

The hairstyling pastes obtained according to Examples 1 to 4 are easy to spread in the hands.

These hairstyling pastes were applied to dry hair which was shaped into the desired hairstyle.

It is noted that the hairstyling pastes give the hair body and texturize it. The hair has, moreover, a clean feel, without any coating effect, and the nongreasy feel. Good fixing of the hairstyle is also obtained.

## Claims

**1.** - Cosmetic composition comprising hydrophobic silica aerogel particles having a specific surface area per unit of mass (SM) ranging from 500 to 1500 $m^2/g$ and one or more hydrophilic fixing polymers, the hydrophobic silica aerogel particles being present in the composition in concentrations ranging from 0.05% to 10% by weight relative to the total weight of the composition.

**2.** - Composition according to the preceding claim, in which the hydrophobic aerogel particles have a specific surface area per unit of mass (SM) ranging from 600 to 1200 $m^2/g$ and better still from 600 to 800 $m^2/g$.

**3.** - Composition according to the preceding claim, in which the hydrophobic aerogel particles have an oil absorption capacity, measured at the wet point, ranging from 5 to 18 ml/g of particles, preferably from 6 to 15 ml/g and better still from 8 to 12 ml/g.

**4.** - Composition according to either one of the preceding claims, **characterized in that** the hydrophobic silica aerogel particles have a size, expressed as the mean diameter D[0.5]), ranging from 1 to 30 $\mu$m, better from 5 to 25 $\mu$m, still better from 5 to 20 $\mu$m and even better still from 5 to 15 $\mu$m.

**5.** - Composition according to any one of the preceding claims, in which the hydrophobic silica aerogel particles are hydrophobic silica particles surface-modified with trimethylsilyl groups.

**6.** - Composition according to any one of the preceding claims, **characterized in that** the hydrophobic silica aerogel particles have a tapped density $\rho$ ranging from 0.04 $g/cm^3$ to 0.10 $g/cm^3$ and preferably from 0.05 $g/cm^3$ to 0.08 $g/cm^3$.

**7.** - Composition according to any one of the preceding claims, **characterized in that** the hydrophobic silica aerogel particles have a specific surface area per unit of volume SV ranging from 5 to 60 $m^2/cm^3$, preferably from 10 to 50 $m^2/cm^3$ and better still from 15 to 40 $m^2/cm^3$.

**8.** - Composition according to any one of the preceding claims, in which the hydrophobic silica aerogel particles are preferably present in the composition in concentrations ranging from 0.1 % to 5% by weight and even more preferentially from 0.5% to 3% by weight relative to the total weight of the composition.

**9.** - Composition according to any one of the preceding claims, in which the hydrophilic fixing polymer is chosen from anionic, cationic, amphoteric and non-ionic fixing polymers, or mixtures thereof.

**10.** - Composition according to any one of the preceding claims, in which the hydrophilic fixing polymer is cationic and preferably chosen from homopolymers or copolymers of acrylic or methacrylic esters or amides containing amine functions, cationic guar gum derivatives, cationic cellulose derivatives, quaternary copolymers of vinylpyrrolidone and of vinylimidazole, and chitosans.

**11.** - Composition according to any one of Claims 1 to 9, **characterized in that** the hydrophilic fixing polymer is anionic

and preferably chosen from copolymers of acrylic and methacrylic acid or salts thereof, crotonic acid copolymers, copolymers of monounsaturated $C_4$-$C_8$ carboxylic acids or anhydrides, polyacrylamides containing carboxylate groups, homopolymers or copolymers containing sulfonic groups, anionic polyurethanes, and anionic grafted silicone polymers.

**12.** - Composition according to any one of Claims 1 to 9, **characterized in that** the hydrophilic fixing polymer is amphoteric and preferably chosen from copolymers containing acidic vinyl units and basic vinyl units, crosslinked and acylated amphoteric polyaminoamides, polymers containing betaine zwitterionic units, chitosan-derived amphoteric polymers, ($C_1$-$C_5$)alkyl vinyl ether/maleic anhydride amphoteric copolymers, amphoteric polyurethanes and amphoteric grafted silicone polymers.

**13.** - Composition according to any one of Claims 1 to 9, **characterized in that** the hydrophilic fixing polymer is non-ionic and preferably chosen from polyalkyloxazolines, vinyl acetate homopolymers and copolymers, homopolymers and copolymers of acrylic esters, copolymers of acrylonitrile, styrene homopolymers and copolymers, polyamides, vinylpyrrolidone homopolymers or copolymers, vinyllactam homopolymers or copolymers, non-ionic polyurethanes, and non-ionic grafted silicone polymers.

**14.** - Composition according to any one of the preceding claims, such that the hydrophilic fixing polymer(s) is (are) present in the composition in a content ranging from 0.1% to 20%, preferably from 0.5% to 10% and better still from 0.5% to 8% by weight relative to the total weight of the composition.

**15.** - Composition according to any one of the preceding claims, **characterized in that** it comprises water in a content ranging from 5% to 98%, preferably from 30% to 95%, better still from 50% to 95% and even better still from 70% to 95% by weight relative to the total weight of the composition.

**16.** - Composition according to any one of the preceding claims, comprising less than 5% of fatty substances, better still less than 3% of fatty substances and even better still no fatty substances.

**17.** - Composition according to any one of the preceding claims, **characterized in that** it is packaged in an aerosol container in the presence of a propellant.

**18.** - Method for the cosmetic treatment of keratin fibres, in particular human keratin fibres such as the hair, **characterized in that** it comprises the step of applying, to said fibres, an effective amount of a composition as defined according to any one of Claims 1 to 17.

**19.** - Use of a composition according to any one of Claims 1 to 17 for hair treatment, especially for treating keratin fibres and in particular for fixing/shaping the hair.

## Patentansprüche

**1.** Kosmetikzusammensetzung, umfassend hydrophobe Siliciumdioxid-Aerogelpartikel mit einer spezifischen Oberfläche pro Masseneinheit (SM) im Bereich von 500 bis 1500 $m^2$/g und ein oder mehrere hydrophile Fixierpolymere, wobei die hydrophoben Siliciumdioxid-Aerogelpartikel in der Zusammensetzung in Konzentrationen im Bereich von 0,05 Gew.% bis 10 Gew.% vorhanden sind, bezogen auf das Gesamtgewicht der Zusammensetzung.

**2.** Zusammensetzung nach einem der vorhergehenden Ansprüche, wobei die hydrophoben Aerogelpartikel eine spezifische Oberfläche pro Masseneinheit (SM) im Bereich von 600 bis 1200 $m^2$/g und besser noch von 600 bis 800 $m^2$/g aufweisen.

**3.** Zusammensetzung nach einem der vorhergehenden Ansprüche, wobei die hydrophoben Aerogelpartikel eine Ölabsorptionskapazität, gemessen am Nasspunkt, im Bereich von 5 bis 18 ml/g der Partikel, vorzugsweise 6 bis 15 ml/g und noch besser 8 bis 12 ml/g aufweisen.

**4.** Zusammensetzung nach einem der vorhergehenden Ansprüche, **dadurch gekennzeichnet, dass** die hydrophoben Siliciumdioxid-Aerogelpartikel eine Größe, ausgedrückt als mittlerer Durchmesser D[0,5]), im Bereich von 1 bis 30 $\mu$m, besser von 5 bis 25 $\mu$m, noch besser 5 bis 20 $\mu$m und sogar noch besser 5 bis 15 $\mu$m aufweisen.

5. Zusammensetzung nach einem der vorhergehenden Ansprüche, wobei die hydrophoben Siliciumdioxid-Aerogelpartikel hydrophobe Siliciumdioxidpartikel sind, die mit Trimethylsilylgruppen oberflächenmodifiziert sind.

6. Zusammensetzung nach einem der vorhergehenden Ansprüche, **dadurch gekennzeichnet, dass** die hydrophoben Siliciumdioxid-Aerogelpartikel eine Stampfdichte $\rho$ im Bereich von 0,04 g/cm$^3$ bis 0,10 g/cm$^3$ und vorzugsweise 0,05 g/cm$^3$ bis 0,08 g/cm$^3$ aufweisen.

7. Zusammensetzung nach einem der vorhergehenden Ansprüche, **dadurch gekennzeichnet, dass** die hydrophoben Siliciumdioxid-Aerogelpartikel eine spezifische Oberfläche pro Volumeneinheit SV im Bereich von 5 bis 60 m$^2$/cm$^3$, vorzugsweise 10 bis 50 m$^2$/cm$^3$ und noch besser 15 bis 40 m$^2$/cm$^3$ aufweisen.

8. Zusammensetzung nach einem der vorhergehenden Ansprüche, wobei die hydrophoben Siliciumdioxid-Aerogelpartikel vorzugsweise in der Zusammensetzung in Konzentrationen im Bereich von 0,1 Gew.% bis 5 Gew.% vorhanden sind und noch bevorzugter 0,5 Gew.% bis 3 Gew.%, bezogen auf das Gesamtgewicht der Zusammensetzung.

9. Zusammensetzung nach einem der vorhergehenden Ansprüche, wobei das hydrophile Fixierpolymer ausgewählt ist aus anionischen, kationischen, amphoteren und nichtionischen Fixierpolymeren oder Mischungen davon.

10. Zusammensetzung nach einem der vorhergehenden Ansprüche, wobei das hydrophile Fixierpolymer kationisch ist und vorzugsweise ausgewählt ist aus Homopolymeren oder Copolymeren von Acryl- oder Methacrylestern oder Amiden, die Aminfunktionen enthalten, kationischen Guargummiderivaten, kationischen Cellulosederivaten, quaternären Copolymeren von Vinylpyrrolidon und von Vinylimidazol, sowie Chitosanen.

11. Zusammensetzung nach einem der Ansprüche 1 bis 9, **dadurch gekennzeichnet, dass** das hydrophile Fixierpolymer anionisch ist und vorzugsweise ausgewählt ist aus Copolymeren von Acryl- und Methacrylsäure oder Salzen davon, Crotonsäurecopolymeren, Copolymeren von einfach ungesättigten $C_4$-$C_8$-Carbonsäuren oder -anhydriden, Polyacrylamiden, die Carboxylatgruppen enthalten, Homopolymeren oder Copolymeren, die Sulfonsäuregruppen enthalten, anionischen Polyurethanen und anionisch gepfropften Silikonpolymeren.

12. Zusammensetzung nach einem der Ansprüche 1 bis 9, **dadurch gekennzeichnet, dass** das hydrophile Fixierpolymer amphoter ist und vorzugsweise ausgewählt ist aus Copolymeren, die saure Vinyleinheiten und basische Vinyleinheiten enthalten, vernetzten und acylierten amphoteren Polyaminoamiden, Polymeren, die zwitterionische Betaineinheiten enthalten, von Chitosan abgeleiteten amphoteren Polymeren, amphoteren ($C_1$-$C_5$)-Alkylvinylether/Maleinsäureanhydrid-Copolymeren, amphoteren Polyurethanen und amphoteren gepfropften Silikonpolymeren.

13. Zusammensetzung nach einem der Ansprüche 1 bis 9, **dadurch gekennzeichnet, dass** das hydrophile Fixierpolymer nichtionisch ist und vorzugsweise ausgewählt ist aus Polyalkyloxazolinen, Vinylacetathomopolymeren und -copolymeren, Homopolymeren und Copolymeren von Acrylestern, Copolymeren von Acrylnitril, Styrolhomopolymeren und -copolymeren, Polyamiden, Vinylpyrrolidonhomopolymeren oder -copolymeren, Vinyllactamhomopolymeren oder -copolymeren, nichtionischen Polyurethanen und nichtionischen gepfropften Silikonpolymeren.

14. Zusammensetzung nach einem der vorhergehenden Ansprüche, wobei das hydrophile Fixierpolymer/die hydrophilen Fixierpolymere in der Zusammensetzung in einem Gehalt im Bereich von 0,1 Gew.% bis 20 Gew.%, bevorzugter 0,5 Gew.% bis 10 Gew.% und noch besser 0,5 Gew.% bis 8 Gew.%, bezogen auf das Gesamtgewicht der Zusammensetzung vorhanden ist bzw. sind.

15. Zusammensetzung nach einem der vorhergehenden Ansprüche, **dadurch gekennzeichnet, dass** sie Wasser in einem Gehalt im Bereich von 5 Gew.% bis 98 Gew.%, vorzugsweise 30 Gew.% bis 95 Gew.%, besser noch 50 Gew.% bis 95 Gew.% und sogar noch besser 70 Gew.% bis 95 Gew.% umfasst, bezogen auf das Gesamtgewicht der Zusammensetzung.

16. Zusammensetzung nach einem der vorhergehenden Ansprüche, umfassend weniger als 5 % fettige Substanzen, besser weniger als 3 % fettige Substanzen und noch besser keine fettigen Substanzen.

17. Zusammensetzung nach einem der vorhergehenden Ansprüche, **dadurch gekennzeichnet, dass** sie in einen Aerosolbehälter in Gegenwart eines Treibmittels verpackt ist.

**18.** Verfahren zur kosmetischen Behandlung von Keratinfasern, insbesondere menschlichen Keratinfasern, wie dem Haar, **dadurch gekennzeichnet, dass** es den Schritt des Aufbringens einer effektiven Menge einer Zusammensetzung wie in einem der Ansprüche 1 bis 17 definiert auf die Fasern umfasst.

**19.** Verwendung einer Zusammensetzung nach einem der Ansprüche 1 bis 17 zur Haarbehandlung, insbesondere zur Behandlung von Keratinfasern und speziell zum Fixieren/Formen des Haares.

**Revendications**

**1.** - Composition cosmétique comprenant des particules d'aérogel de silice hydrophobe présentant une surface spécifique par unité de masse (SM) allant de 500 à 1500 $m^2/g$, et un ou plusieurs polymères fixants hydrophiles, les particules d'aérogel de silice hydrophobe étant présentes dans la composition dans des concentrations allant de 0,05 à 10% en poids par rapport au poids total de la composition.

**2.** - Composition selon la revendication précédente, dans laquelle les particules d'aérogel hydrophobe présentent une surface spécifique par unité de masse (SM) allant de 600 à 1200 $m^2/g$ et mieux de 600 à 800 $m^2/g$. et une taille exprimée en diamètre moyen (D[0,5]) allant de 1 à 30 μm et/ou une capacité d'absorption d'huile mesurée au WET POINT allant de 5 à 18 ml/g de particules, de préférence de 6 à 15 ml/g et mieux de 8 à 12 ml/g.

**3.** - Composition selon la revendication précédente, dans laquelle les particules d'aérogel hydrophobe présentent une capacité d'absorption d'huile mesurée au wet point allant de 5 à 18 ml/g de particules, de préférence de 6 à 15 ml/g et mieux de 8 à 12 ml/g.

**4.** - Composition selon l'une quelconque des revendications précédentes, **caractérisée en ce que** les particules d'aérogel de silice hydrophobe présentent une taille exprimée en diamètre moyen D[0.5]) allant de 1 à 30 μm, mieux de 5 à 25 μm, encore mieux de 5 à 20 μm et même encore mieux de 5 à 15 μm.

**5.** - Composition selon l'une quelconque des revendications précédentes, dans laquelle les particules d'aérogel de silice hydrophobe sont des particules de silice hydrophobe modifiée en surface par des groupements triméthylsilyle.

**6.** - Composition selon l'une quelconque des revendications précédentes, **caractérisée en ce que** les particules d'aérogel de silice hydrophobe présentent une densité tassée p allant de 0,04 $g/cm^3$ à 0,10 $g/cm^3$, de préférence de 0,05 $g/cm^3$ à 0,08 $g/cm^3$.

**7.** - Composition selon l'une quelconque des revendications précédentes, **caractérisée en ce que** les particules d'aérogel de silice hydrophobe présentent une surface spécifique par unité de volume SV allant de 5 à 60 $m^2/cm^3$, de préférence de 10 à 50 $m^2/cm^3$ et mieux de 15 à 40 $m^2/cm^3$.

**8.** - Composition selon l'une quelconque des revendications précédentes, dans laquelle les particules d'aérogel de silice hydrophobe sont de préférence présentes dans la composition dans des concentrations allant de 0,1 à 5% en poids et, encore plus préférentiellement de 0,5 à 3% en poids par rapport au poids total de la composition.

**9.** - Composition selon l'une quelconque des revendications précédentes, dans laquelle le polymère fixant hydrophile est choisi parmi les polymères fixants anioniques, cationiques, amphotères, non-ioniques, ou leurs mélanges.

**10.** - Composition selon l'une quelconque des revendications précédentes, dans laquelle le polymère fixant hydrophile est cationique et de préférence choisi parmi les homopolymères ou copolymères d'esters ou d'amides acryliques ou méthacryliques à fonctions aminées, les dérivés de gomme de guar cationiques, les dérivés de cellulose cationiques, les copolymères quaternaires de vinylpyrrolidone et de vinylimidazole, et les chitosanes.

**11.** - Composition selon l'une quelconque des revendications 1 à 9, **caractérisée en ce que** le polymère fixant hydrophile est anionique et de préférence choisi parmi les copolymères d'acide acrylique et méthacrylique ou leurs sels, les copolymères d'acide crotonique, les copolymères d'acides ou d'anhydrides carboxyliques monoinsaturés en $C_4$-$C_8$, les polyacrylamides à groupements carboxylate, les homopolymères ou copolymères à groupes sulfoniques, les polyuréthanes anioniques, et les polymères siliconés greffés anioniques.

**12.** - Composition selon l'une quelconque des revendications 1 à 9, **caractérisée en ce que** le polymère fixant hydrophile

est amphotère et de préférence choisi parmi les copolymères à motifs vinyliques acides et à motifs vinyliques basiques, les polyaminoamides amphotères réticulés et acylés, les polymères à motifs zwittérioniques bétaïniques, les polymères amphotères dérivés du chitosane, les copolymères amphotères alkyl($C_1$-$C_5$)vinyléther/anhydride maléique, les polyuréthanes amphotères et les polymères siliconés greffés amphotères.

**13.** - Composition selon l'une quelconque des revendications 1 à 9, **caractérisée en ce que** le polymère fixant hydrophile est non ionique et de préférence choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolyméres ou copolymères de vinylpyrrolidone, les homopolyméres ou copolymères de vinyllactame, les polyuréthanes non ioniques, et les polymères siliconés greffés non ioniques.

**14.** - Composition selon l'une quelconque des revendications précédentes telle que le ou les polymères fixants hydrophiles sont présents dans la composition dans une teneur allant de 0,1 à 20%, de préférence de 0,5 à 10%, mieux de 0,5 à 8% en poids par rapport au poids total de la composition.

**15.** - Composition selon l'une quelconque des revendications précédentes, **caractérisée en ce qu'**elle comprend de l'eau dans une teneur allant de de 5 à 98%, de préférence de 30 à 95%, mieux de 50 à 95%, encore mieux de 70 à 95 % en poids par rapport au poids total de la composition.

**16.** - Composition selon l'une quelconque des revendications précédentes, comprenant moins de 5% de corps gras, mieux moins de 3% de corps gras, encore mieux pas de corps gras.

**17.** - Composition selon l'une quelconque des revendications précédentes, **caractérisée en ce qu'**elle conditionnée dans un récipient aérosol en présence d'un agent propulseur.

**18.** - Procédé de traitement cosmétique des fibres kératiniques, en particulier des fibres kératiniques humaines telles que les cheveux, **caractérisé en ce qu'**il consiste à appliquer sur lesdites fibres une quantité efficace d'une composition telle que définie selon l'une quelconque des revendications 1 à 17.

**19.** - Utilisation d'une composition selon l'une quelconque des revendications 1 à 17 pour le traitement capillaire, notamment pour le traitement des fibres kératiniques et en particulier pour la fixation/la mise en forme des cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007051511 A1
- WO 2009059869 A2
- US 7470725 B
- FR 1222944
- DE 2330956
- LU 75370
- LU 75371
- FR 1580545
- FR 2265782
- FR 2265781
- FR 1564110
- FR 2439798
- US 2047398 A
- US 2723248 A
- US 2102113 A
- GB 839805 A
- FR 2350384
- FR 2357241
- FR 2198719
- US 4128631 A
- US 3734874 A
- US 3779993 A
- US 4119680 A
- US 4300580 A
- US 4973656 A
- US 5660816 A
- US 5662893 A
- US 5674479 A
- WO 9518191 A
- WO 9708261 A
- WO 9720899 A
- FR 2077143
- FR 2393573
- US 3589578 A
- US 4031307 A
- US 4131576 A
- US 3836537 A
- FR 1400366
- EP 0412704 A
- EP 0412707 A
- EP 0640105 A
- WO 9500578 A
- EP 0582152 A
- WO 9323009 A
- US 4693935 A
- US 4728571 A
- US 4972037 A
- EP 0751162 A
- EP 0637600 A
- EP 0648485 A
- FR 2743297
- EP 0656021 A
- WO 9403510 A
- EP 0619111 A


### Non-patent literature cited in the description


- **BRINKER C.J. ; SCHERER G.W.** Sol-Gel Science. Academic Press, 1990
- *The Journal of the American Chemical Society,* February 1938, vol. 60, 309
- **VAN DE HULST, H.C.** Light Scattering by Small Particles. Wiley, 1957